# EUROPEAN PATENT APPLICATION

(11) **EP 4 297 038 A1**
(43) Date of publication of application: **27.12.2023**
(21) Application number: 23179154.2
(22) Date of filing: 14.06.2023
(51) Int. Cl.: G16H 10/20, G16H 15/00, G16H 30/40, G16H 50/70

(54) **METHOD AND APPARATUS FOR ANALYZING BIOMARKERS**

(30) Priority: 15.06.2022 KR 20220073072; 11.07.2022 KR 20220085305; 21.03.2023 KR 20230036368
(71) Applicant: Lunit Inc., Seoul 06241 (KR)
(72) Inventor: PAENG, Kyung Hyun, 07342 Seoul (KR); OH, Seung Yun, 03612 Seoul (KR); MOON, Ji Min, 05689 Seoul (KR); KIM, Se Jin, 13157 Gyeonggi-do (KR)
(74) Representative: AWA Sweden AB

(57) **Abstract**

A computing apparatus includes at least one memory storing at least one program, and at least one processor configured to, by executing the at least one program, acquire at least one of first information regarding a primary clinical trial previously performed on a certain drug and second information indicating an association between the drug and each of candidate biomarkers, set a criterion related to responsitivity to the drug based on the acquired information, and generate information related to a secondary clinical trial based on the set criterion.

## Description

### BACKGROUND

### 1. Field

The present disclosure relates to a method and apparatus for analyzing a biomarker for a clinical trial.

### 2. Description of the Related Art

Companion diagnostics (CDx) is a diagnostic method or test performed to cure or treat patients by using drugs, and is commonly developed jointly together with new drugs (i.e., newly developed drugs) during clinical trials. When clinical trials are conducted for the development of new drugs, it is significant to select appropriate subjects and allow the selected subjects to participate in the clinical trials in order to increase the success rate of the clinical trials. To this end, a method of selecting subjects to participate in clinical trials via biomarkers may be used.

### SUMMARY

Provided are a method and apparatus for analyzing a biomarker. Provided are a computer-readable recording medium having recorded thereon a program for executing the method in a computer. The technical problems to be solved are not limited to the technical problems as described above, and other technical problems may be present.

Additional aspects will be set forth in part in the description which follows and, in part, will be apparent from the description, or may be learned by practice of the presented embodiments of the disclosure.

According to an aspect of an embodiment, a computing apparatus includes at least one memory storing at least one program, and at least one processor configured to, by executing the at least one program, acquire at least one of first information regarding a primary clinical trial previously performed on a certain drug and second information indicating an association between the drug and each of candidate biomarkers, set a criterion related to responsitivity to the drug based on the acquired information, and generate information related to a secondary clinical trial based on the set criterion.

According to an aspect of another embodiment, a method of analyzing a biomarker includes acquiring at least one of first information regarding a primary clinical trial previously performed on a certain drug and second information indicating an association between the drug and each of candidate biomarkers, setting a criterion related to responsitivity to the drug based on the acquired information, and generating information related to a secondary clinical trial based on the set criterion.

According to an aspect of another embodiment, a computer-readable recording medium includes a recording medium having recorded thereon a program for executing the method described above in a computer.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a view illustrating an example of a system for analyzing a biomarker, according to an embodiment;
FIG. 2 is a block diagram illustrating an example of a system and a network for analyzing a biomarker by using machine learning, according to an embodiment;
FIG. 3A is a block diagram illustrating an example of a user terminal according to an embodiment;
FIG. 3B is a block diagram illustrating an example of a server according to an embodiment;
FIG. 4 is a flowchart illustrating an example of a method of analyzing a biomarker, according to an embodiment;
FIG. 5 is a diagram illustrating an example of first information according to an embodiment;
FIG. 6 is a view illustrating an example of second information according to an embodiment;
FIG. 7 is a flowchart illustrating an example in which a processor operates, according to an embodiment;
FIG. 8 is a view illustrating an example in which a processor operates, according to an embodiment;
FIG. 9 is a flowchart illustrating another example of a method of analyzing a biomarker, according to an embodiment;
FIG. 10 is a flowchart illustrating another example of a method of analyzing a biomarker, according to an embodiment;
FIG. 11 is a diagram illustrating an example in which a processor operates, according to an embodiment;
FIG. 12 is a diagram illustrating another example in which a processor operates, according to an embodiment;
FIG. 13 is a diagram illustrating another example in which a processor operates, according to an embodiment; and
FIG. 14 is a diagram illustrating another example in which a processor operates, according to an embodiment.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

Regarding the terms in embodiments, the general terms which are currently and widely used are selected, but meanings of the terms may be changed according to intention, a judicial precedence, the appearance of a new technology, and the like. In addition, in certain cases, terms may be arbitrarily selected by the applicant. In such a case, the meaning of the terms will be described in detail at the corresponding portion in the description of the present disclosure. Therefore, the terms used herein should be defined based on the meaning of the terms and the descriptions provided herein, rather than just the names of the terms.

Unless explicitly described to the contrary, the word "comprise" and variations such as "comprises" or "comprising" will be understood to imply the inclusion of stated elements but not the exclusion of any other elements. In addition, the terms "- unit", "- module", and the like described herein mean units for processing at least one function or operation, and may be implemented by hardware components, software components, or combinations thereof.

Although the terms first, second, etc. may be used herein to describe various elements or components, these elements or components should not be limited by these terms. These terms may be only used to distinguish one element or component from another element or component.

According to an embodiment, a "pathological slide image" may refer to an image obtained by photographing a pathological slide that is fixed and stained through a series of chemical treatment processes for a tissue or the like removed from the human body. In addition, the pathological slide image may refer to a whole slide image (WSI) including a high-resolution image of a whole slide, and may refer to a portion of the WSI, for example, one or more patches. For example, the pathological slide image may refer to a digital image photographed or scanned via a scan apparatus (e.g., a digital scanner), and may include information regarding a specific protein, cell, tissue, and/or structure in the human body. In addition, the pathological image may include one or more patches, and histological information may be applied (e.g., tagged) to the one or more patches via an annotation operation.

According to an embodiment, "medical information" may refer to any medically meaningful information that may be extracted from a medical image, and may include, for example, the area, location, and size of a tumor cell within the medical image, cancer diagnosis information, information associated with the possibility of developing cancer of a subject, and/or medical conclusions associated with cancer treatment, and the like, but is not limited thereto. In addition, the medical information may include quantified numerical values that may be obtained from the medical image, as well as information obtained by visualizing numerical values, prediction information according to the numerical values, image information, statistical information, and the like. The medical information generated as described above may be provided to a user terminal or output or transmitted to a display apparatus to be displayed.

Hereinafter, embodiments will be described in detail with reference to the accompanying drawings. However, the embodiments may be implemented in various forms and are not limited to examples described herein.

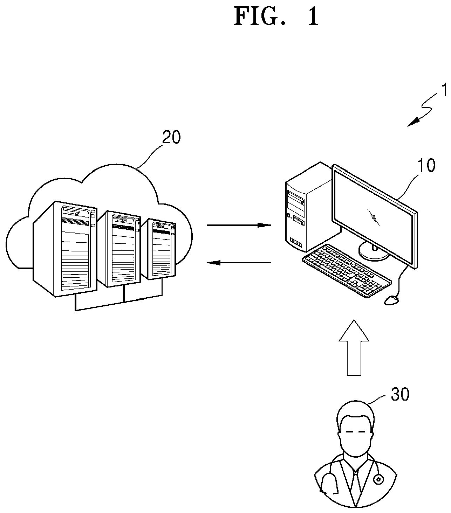

FIG. 1 is a view illustrating an example of a system for analyzing a biomarker, according to an embodiment.

Referring to FIG. 1, a system 1 may include a user terminal 10 and a server 20. For example, the user terminal 10 and the server 20 may be connected to each other by a wired or wireless communication method to transmit and receive data (e.g., image data, or the like) to and from each other.

For convenience of description, FIG. 1 illustrates that the system 1 includes the user terminal 10 and the server 20, but the present disclosure is not limited thereto. For example, the system 1 may include another external device (not shown), and operations of the user terminal 10 and the server 20 described below may be implemented by a single device (e.g., the user terminal 10 or the server 20) or more devices.

The user terminal 10 may be a computing apparatus that is provided with a display apparatus and a device (e.g., a keyboard, a mouse, or the like) for receiving a user input and includes a memory and a processor. For example, the user terminal 10 may correspond to a notebook personal computer (PC), a desktop PC, a laptop computer, a tablet computer, a smartphone, or the like, but is not limited thereto.

The server 20 may be an apparatus that communicates with an external device (not shown) including the user terminal 10. For example, the server 20 may be an apparatus that stores various types of data including a pathological slide image, a bitmap image corresponding to the pathological slide image, information generated by analyzing the pathological slide image (e.g., including information regarding at least one tissue and cell expressed in the pathological slide image, biomarker expression information, and the like), information regarding a clinical trial (including information regarding the result of the clinical trial), information regarding a plurality of biomarkers, and information regarding mechanisms of a plurality of drugs. Alternatively, the server 20 may be a computing apparatus that includes a memory and a processor and has a self-computation capability. When the server 20 is the computing apparatus, the server 20 may perform at least some of operations of the user terminal 10 described below with reference to FIGS. 1 to 14. For example, the server 20 may also be a cloud server, but is not limited thereto.

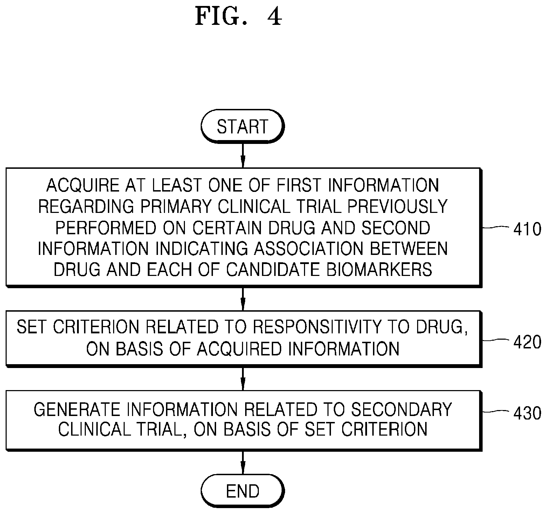

The user terminal 10 may output a pathological slide image and/or an image indicating information generated by analyzing a pathological slide. As an example, various types of information regarding at least one tissue and cell expressed in the pathological slide image may be expressed in the image. In addition, expression information of a biomarker may be expressed in the image. As another example, the image may be a report including information related to a clinical trial. For example, the report may include information regarding a subject who is subject to the clinical trial and/or information related to at least one biomarker to be used for design of the clinical trial. An example of a report that may be output by the user terminal 10 is described below with reference to FIGS. 4 to 14.

The pathological slide image may refer to an image obtained by photographing a pathological slide that is fixed and stained through a series of chemical treatment processes to observe a tissue or the like removed from a human body under a microscope. As an example, the pathological slide image may refer to a WSI including a high-resolution image of a whole slide. As another example, the pathological slide image may refer to a portion of a high-resolution WSI.

The pathological slide image may be obtained from a stained tissue sample of a subject. For example, the tissue sample may be stained in various staining methods, such as hematoxylin and eosin, trichrome, periodic acid schiff, autoradiogrphy, enzyme histochemistry, immuno-fluorescence, and immunohistochemistry. The stained tissue sample may be used for histology and biopsy evaluations, and thus may be a basis for determining whether or not to move on to molecular profile analysis to understand a disease state.

The pathological slide image may refer to a patch region that is segmented in units of patches from the WSI. For example, a patch may have a certain region size. Alternatively, the patch may refer to a region including each of objects included in the whole slide. In addition, the pathological slide image may refer to a digital image captured by using a microscope, and may include information regarding a cell, tissue, and/or structure within a human body.

Meanwhile, a clinical trial may refer to a test or study conducted on a human to prove the safety and effect of a new drug, a medical device, a new procedure or surgical method, or the like. The clinical trial may be usually divided into four phases, a phase 1 trial may be a process of identifying the most appropriate method and dose of a new treatment, a phase 2 trial may be a process of proving the efficacy and safety of the new treatment, a phase 3 trial may be a process of establishing the efficacy of the new treatment to some extent and then comparing the new treatment with an existing treatment, and a phase 4 trial may be a process of reviewing the safety, long-term side effects, and the like that are not identified in the post-marketing phase 3 trial and conducting additional research. Trials or studies may be conducted through several phases even within a clinical trial of the same phase.

A clinical trial for a drug may be conducted several times, and selecting, on the basis of survival data, a responder or a non-responder from among subjects who participate in the clinical trial may be significant. For example, the survival data may include administration responsitivity of a subject, an actual survival time of the subject, and a change in the size of a cancer tissue of the subject, and the like, and the survival data may be acquired by analyzing a pathological slide image.

The system 1 according to an embodiment may select appropriate subjects via machine learning-based analysis according to phases of a clinical trial. For example, the system 1 may set a criterion related to responsitivity to a drug as a criterion for selecting subjects. Here, the system 1 may set the criterion described above, on the basis of at least one of first information regarding a previously performed primary clinical trial and second information indicating an association between a drug and each of candidate biomarkers. In addition, the system 1 may generate, on the basis of the set criterion, information related to a secondary clinical trial.

In the present disclosure, the primary clinical trial and the secondary clinical trial may be terms used to describe trials or studies conducted back and forth in time series, and may be used to distinguish one clinical trial from another clinical trial. In the present disclosure, the primary clinical trial and the secondary clinical trial may be clinical trials of the same phase or clinical trials of different phases.

In detail, after the primary clinical trial is completed, the system 1 may use, as a dataset, various types of information (e.g., a tissue, an image, and the like) acquired during the primary clinical trial, and responsivity to and treatment results from the drug. The system 1 may detect biological features from a pathological slide image of a subject who participates in the primary clinical trial, and may analyze a dataset by using the biological features. For example, the system 1 may analyze the dataset by using a machine learning model that derives medical information from the pathological slide image. The analyzed dataset may be expressed or quantified in various forms, and association analysis with the treatment result of a subject may be conducted via the expressed or quantified dataset. In other words, the system 1 may identify, on the basis of the dataset, a feature via which the responsitivity to the drug may be predicted, and may select, on the basis of the identified feature, at least one subject to constitute a patient group (or a subject group) for the secondary clinical trial.

In other words, the system 1 may generate information for assisting design of the secondary clinical trial, on the basis of the criterion established on the basis of the result of the primary clinical trial, and thus, not only the improvement of a success rate of clinical trials, but also the approval of companion diagnostics (CDx) may be promoted.

Meanwhile, for convenience of description, as described herein, the user terminal 10 may establish the criterion related to the responsitivity to the drug, on the basis of the first information regarding the previously performed primary clinical trial and the second information indicating the association between the drug and each of the candidate biomarkers, and may generate the information related to the secondary clinical trial on the basis of the established criterion, but is not limited thereto. For example, at least some of operations performed by the user terminal 10 may also be performed by the server 20.

In other words, at least some of operations of the user terminal 10 described with reference to FIGS. 1 to 14 may be performed by the server 20. For example, the server 20 may acquire the first information and the second information from an external device including the user terminal 10. Also, the server 20 may establish the criterion related to the responsitivity to the drug, and may generate the information related to the secondary clinical trial. In addition, the server 20 may transmit, to the user terminal 10, the generated information and/or the report created on the basis of the generated information. However, the operation of the server 20 is not limited to the operation described above.

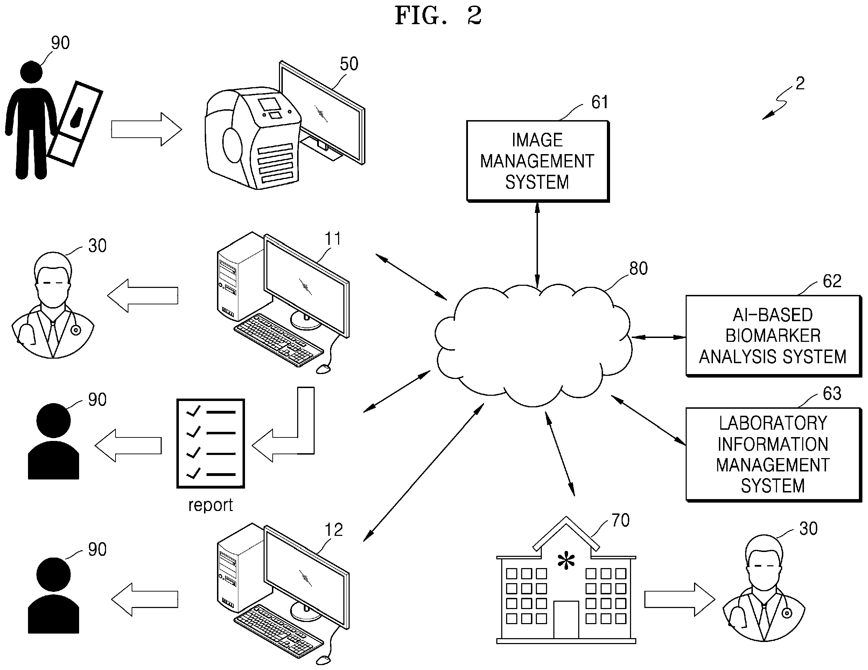

FIG. 2 is a block diagram illustrating an example of a system and a network for analyzing a biomarker by using machine learning, according to an embodiment.

Referring to FIG. 2, a system 2 may include user terminals 11 and 12, a scanner 50, an image management system 61, an artificial intelligence (Al)-based biomarker analysis system 62, a laboratory information management system 63, and a hospital or laboratory server 70. In addition, components (e.g., the user terminals 11 and 12, the scanner 50, the image management system 61, the Al-based biomarker analysis system 62, the laboratory information management system 63, and the hospital or laboratory server 70) included in the system 2 may be connected to one other via a network 80. For example, the network 80 may be a network via which the components (e.g., the user terminals 11 and 12, the scanner 50, the image management system 61, the Al-based biomarker analysis system 62, the laboratory information management system 63, and the hospital or laboratory server 70) may be connected to one other in a wired or wireless communication method. For example, the system 2 illustrated in FIG. 2 may include a network that may be connected to servers in hospitals, laboratories, and the like, and/or user terminals of doctors or researchers.

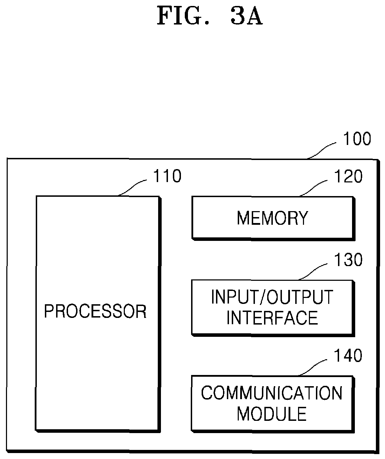

According to various embodiments of the present disclosure, a method described below with reference to FIGS. 3A to 14 may be performed by the user terminals 11 and 12, the image management system 61, the Al-based biomarker analysis system 62, the laboratory information management system 63, and/or the hospital or laboratory server 70.

The scanner 50 may acquire a digitized image from a tissue sample slide generated by using a tissue sample of a subject 90. For example, each of the scanner 50, the user terminals 11 and 12, the image management system 61, the Al-based biomarker analysis system 62, the laboratory information management system 63, and/or the hospital or laboratory server 70 may be connected to the network 80, such as the Internet, via one or more computers, servers, and/or mobile devices, or may communicate with a user 30 and/or the subject 90 via one or more computers, and/or mobile devices.

The user terminals 11 and 12, the image management system 61, the Al-based biomarker analysis system 62, the laboratory information management system 63, and/or the hospital or laboratory server 70 may generate tissue samples of one or more subjects 90, tissue sample slides, digitized images of the tissue sample slides, or any combination thereof, and otherwise, may acquire the same from another apparatus. In addition, the user terminals 11 and 12, the image management system 61, the Al-based biomarker analysis system 62, and the laboratory information management system 63 may acquire any combination of pieces of subject-specific information, such as age, medical history, cancer treatment history, family history, past biopsy records of the subject 90, or disease information of the subject 90.

The scanner 50, the user terminals 11 and 12, the image management system 61, the laboratory information management system 63, and/or the hospital or laboratory server 70 may transmit the digitized slide images and/or the subject-specific information to the Al-based biomarker analysis system 62 via the network 80. The Al-based biomarker analysis system 62 may include one or more storage devices (not shown) for storing images and data received from at least one of the scanner 50, the user terminals 11 and 12, the image management system 61, the laboratory information management system 63, and/or the hospital or laboratory server 70. In addition, the Al-based biomarker analysis system 62 may include a machine learning model storage that stores a machine learning model trained to process the received images and data. For example, the Al-based biomarker analysis system 62 may include a machine learning model that is learned and trained to predict, from a pathological slide image of the subject 90, at least one of information regarding at least one cell, information regarding at least one region, information related to a biomarker, medical diagnosis information, and/or medical treatment information.

The scanner 50, the user terminals 11 and 12, the Al-based biomarker analysis system 62, the laboratory information management system 63, and/or the hospital or laboratory server 70 may transmit the digitized slide images, the subject-specific information, and/or the results of analyzing the digitized slide images to the image management system 61 via the network 80. The image management system 61 may include a storage for storing the received images and a storage for storing the analysis results.

In addition, according to various embodiments of the present disclosure, the machine learning model, which is learned and trained to predict, from the pathological slide image of the subject 90, at least one of the information regarding at least one cell, the information regarding at least one region, the information related to the biomarker, the medical diagnosis information, and/or the medical treatment information, may be stored and operated in the user terminals 11 and 12 and/or the image management system 61.

According to various embodiments of the present disclosure, a method of analyzing a pathological slide image, a method of processing subject information, a method of selecting a subject group, a method of designing a clinical trial, a method of generating biomarker expression information, and/or a method of setting a reference value for a particular biomarker may be performed not only by the Al-based biomarker analysis system 62, but also by the user terminals 11 and 12, the image management system 61, the laboratory information management system 63, and/or the hospital or laboratory server 70.

FIG. 3A is a block diagram illustrating an example of a user terminal according to an embodiment.

Referring to FIG. 3A, a user terminal 100 may include a processor 110, a memory 120, an input/output interface 130, and a communication module 140. For convenience of description, FIG. 3A illustrates only components related to the present disclosure. Accordingly, the user terminal 100 may further include other general-purpose components, in addition to the components illustrated in FIG. 3A. In addition, it may be obvious to those skilled in the art that the processor 110, the memory 120, the input/output interface 130, and the communication module 140 illustrated in FIG. 3A may be implemented as independent devices.

In addition, an operation of the user terminal 100 may be performed by the user terminals 11 and 12, the image management system 61, the Al-based biomarker analysis system 62, the laboratory information management system 63, and/or the hospital or laboratory server 70 illustrated in FIG. 2.

The processor 110 may process instructions of a computer program by performing basic arithmetic, logic, and input/output operations. Here, the instructions may be provided from the memory 120 or an external apparatus (e.g., the server 20 or the like). In addition, the processor 110 may control overall operations of the other components included in the user terminal 100.

The processor 110 may acquire at least one of first information regarding a primary clinical trial previously performed on a certain drug, and second information indicating an association between the drug and each of candidate biomarkers. In addition, the processor 110 may set, on the basis of the acquired information, a criterion related to responsitivity to the drug. In addition, the processor 110 may generate, on the basis of the set criterion, information related to a secondary clinical trial.

For example, the first information and/or the second information may be acquired from the memory 120 or an external device. Here, the external device may be at least one device included in the system 2 illustrated in FIG. 2.

The first information may include a pathological slide image of each of subjects participating in the primary clinical trial. Also, the association included in the second information may be determined by at least one of an expression level of at least one genome related to a mechanism of the drug in cancer cell, a relationship between expression locations of candidate biomarkers and a location at which the drug acts, and genome oncogenic addiction related to the cancer cell.

In addition, the processor 110 may determine at least one of at least one biomarker from among the candidate biomarkers and a cut-off value corresponding to the at least one biomarker. Here, the determined factor (i.e., the at least one biomarker or the cut-off value) may be used as a criterion for distinguishing between a responder and a non-responder to the drug from among subjects of the primary clinical trial.

In addition, the information related to the secondary clinical trial may include at least one of information regarding at least one subject who is subject to the secondary clinical trial and information related to at least one biomarker to be used for design of the secondary clinical trial. Here, the information related to the at least one biomarker may include at least one of an identifier of the at least one biomarker, a cut-off value corresponding to the at least one biomarker, predictive power information related to responsitivity of the at least one biomarker to the drug, a list in which the at least one biomarker is arranged by a certain criterion, and a relationship between the at least one biomarker and the result of the primary clinical trial.

Here, the identifier may include various types of information capable of specifying a biomarker. For example, the identifier may include any characters, numbers, or other marks that may specify a biomarker, such as a name, a symbol, or a serial number.

In addition, the processor 110 may acquire, for each of subjects participating in the primary clinical trial, a pathological slide image indicating before administration of the drug and a pathological slide image indicating after the administration of the drug. Also, the processor 110 may set a criterion related to responsitivity to the drug by further considering changes in the acquired pathological slide images.

In addition, the processor 110 may output a report including the information related to the secondary clinical trial.

The processor 110 may be implemented as an array of a plurality of logical gates, or may be implemented as a combination of a general-purpose microprocessor and a memory that stores a program that may be executed by the general-purpose microprocessor. For example, the processor 110 may include a general-purpose processor, a central processing unit (CPU), a microprocessor, a digital signal processor (DSP), a controller, a microcontroller, a state machine, and the like. In some environments, the processor 110 may also include an application-specific integrated circuit (ASIC), a programmable logic device (PLD), a field programmable gate array (FPGA), and the like. For example, the processor 110 may refer to a combination of processing devices, such as a combination of a DSP and a microprocessor, a combination of a plurality of microprocessors, a combination of one or more microprocessors combined with a DSP core, or a combination of any other components.

The memory 120 may include any non-transitory computer-readable recording medium. For example, the memory 120 may include a permanent mass storage device, such as random access memory (RAM), read-only memory (ROM), a disk drive, a solid state drive (SSD), or flash memory. As another example, the permanent mass storage device, such as ROM, an SSD, flash memory, or a disk drive, may be a separate permanent storage device that is distinguished from a memory. In addition, the memory 120 may store an operating system (OS) and at least one program code (e.g., code for the processor 110 to perform an operation to be described below with reference to FIGS. 4 to 14).

Software components as described above may be loaded from a computer-readable recording medium separate from the memory 120. The separate computer-readable recording medium may be a recording medium that may be directly connected to the user terminal 100, for example, may include a computer-readable recording medium, such as a floppy drive, a disk, a tape, a DVD/CD-ROM drive, a memory card, or the like. Alternatively, the software components may be loaded into the memory 120 via the communication module 140, rather than into a computer-readable recording medium. For example, at least one program may be loaded into the memory 120 on the basis of a computer program installed by files provided via the communication module 140 by developers or a file distribution system that distributes installation files of applications (e.g., a computer program for the processor 110 to perform an operation described below with reference to FIGS. 4 to 14).

The input/output interface 130 may be a unit for an interface with a device (e.g., a keyboard, a mouse, or the like) for an input or output, which may be connected to the user terminal 100 or included in the user terminal 100. Although FIG. 3A illustrates the input/output interface 130 as a component configured separately from the processor 110, the input/output interface 130 is not limited thereto and may be configured to be included in the processor 110.

The communication module 140 may provide a component or function for the server 20 of FIG. 1 and the user terminal 100 to communicate with each other via a network. In addition, the communication module 140 may provide a component or function for the user terminal 100 to communicate with another external device. For example, a control signal, a command, data, and the like provided under control of the processor 110 may be transmitted to the server 20 of FIG. 1 and/or the external device via the communication module 140 and the network.

Meanwhile, although not illustrated in FIG. 3A, the user terminal 100 may further include a display apparatus. Alternatively, the user terminal 100 may be connected to an independent display apparatus in a wired or wireless communication method to transmit and receive data to and from the independent display apparatus. For example, a pathological slide image, analysis information of the pathological slide image, biomarker expression information, biomarker analysis information, a report, and the like may be provided to the user 30 of FIG. 2 via the display apparatus.

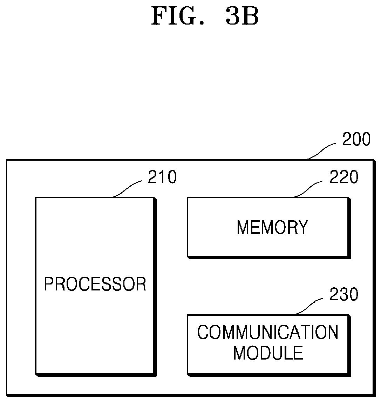

FIG. 3B is a block diagram illustrating an example of a server according to an embodiment.

Referring to FIG. 3B, a server 200 may include a processor 210, a memory 220, and a communication module 230. For convenience of description, FIG. 3B illustrates only components related to the present disclosure. Accordingly, the server 200 may further include other general-purpose components, in addition to the components illustrated in FIG. 3B. In addition, it may be obvious to those skilled in the art that the processor 210, the memory 220, and the communication module 230 illustrated in FIG. 3B may be implemented as independent apparatuses.

The processor 210 may acquire first information and/or second information from at least one of the memory 220, the user terminal 10 of FIG. 1, and an external device. The processor 210 may set, on the basis of acquired information, a criterion related to responsitivity to a drug, or may generate information related to a secondary clinical trial on the basis of the set criterion. Alternatively, the processor 210 may acquire, for each of subjects participating in a primary clinical trial, pathological slide images indicating before and after administration of the drug, and may set the criterion related to the responsitivity to the drug by further considering changes in the pathological slide images. Alternatively, the processor 210 may generate a report including information related to the secondary clinical trial.

In other words, at least one of the operations of the processor 110 described above with reference to FIG. 3A may be performed by the processor 210. Here, the user terminal 100 of FIG. 3A may output, via a display apparatus, information transmitted from the server 200.

Meanwhile, an implementation example of the processor 210 is the same as the implementation example of the processor 110 described above with reference to FIG. 3A, and thus, a detailed description thereof is omitted.

The memory 220 may store various types of data, such as a pathological slide image and data generated according to an operation of the processor 210. In addition, the memory 220 may store an OS and at least one program (e.g., a program needed for the processor 210 to operate).

Meanwhile, an implementation example of the memory 220 is the same as the implementation example of the memory 120 described above with reference to FIG. 3A, and thus, a detailed description thereof is omitted.

The communication module 230 may provide a component or function for the server 200 and the user terminal 100 of FIG. 3A to communicate with each other via a network. In addition, the communication module 230 may provide a component or function for the server 200 to communicate with anther external device. For example, a control signal, a command, data, and the like provided under control of the processor 210 may be transmitted to the user terminal 100 of FIG. 3A and/or an external device via the communication module 230 and the network.

FIG. 4 is a flowchart illustrating an example of a method of analyzing a biomarker, according to an embodiment.

Referring to FIG. 4, a method of analyzing a biomarker may include operations processed in time series by the user terminal 10 or 100 of FIG. 1 or 3A, or the processor 110 of 3A. Therefore, even when the above description of the user terminal 10 or 100 of FIG. 1 or 3A or the processor 110 of 3A is omitted below, the description may also be applied to the method of analyzing a biomarker, illustrated in FIG. 4.

In addition, as described above with reference to FIGS. 1 and 3B, at least one of the operations of the flowchart illustrated in FIG. 4 may be processed by the server 20 or 200 or the processor 210.

In operation 410, the processor 110 may acquire at least one of first information regarding a primary clinical trial previously performed on a certain drug, and second information indicating an association between the drug and each of candidate biomarkers.

After the primary clinical trial on the drug is completed, the processor 110 may acquire at least one of the first information and the second information. For example, the first information may be a dataset including the result of the primary clinical trial, and the dataset may include survival data of subjects and/or pathological slide images of the subjects. The survival data may be acquired by analyzing the pathological slide images, may be acquired by measuring actual survival times of the subjects after the primary clinical trial, may be acquired by analyzing changes in the size of a cancer tissue before and after administration of the drug, or may be acquired by analyzing responsitivities of the subjects to the administration of drug by an invasive or non-invasive method. For example, an association included in the second information may be determined by at least one of an expression level of at least one genome related to a mechanism of the drug in a cancer cell, a relationship between expression locations of candidate biomarkers and a location at which the drug acts, and genome oncogenic addiction related to the cancer cell.

For example, the processor 110 may receive the first information and/or the second information from the memory 120 or an external device, or the processor 110 may generate the first information and/or the second information.

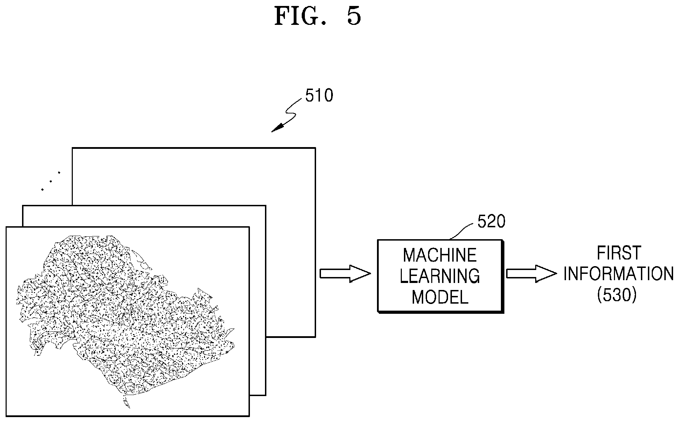

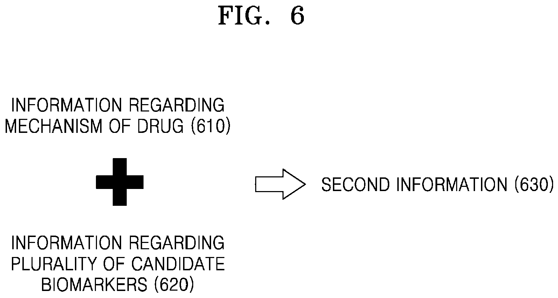

Hereinafter, examples of the first information and the second information are described with reference to FIGS. 5 and 6.

FIG. 5 is a diagram illustrating an example of first information according to an embodiment.

First information 530 may include a dataset indicating the result of a primary clinical trial and/or pathological slide images 510 of subjects. For example, the result of the primary clinical trial may be derived as survival data of a subject, and the survival data may include responsitivity to drug administration, a survival time after the drug administration, changes in the size of a cancer tissue before and after the drug administration, and the like. Accordingly, the survival data may distinguish a responder and a non-responder to the drug.

Meanwhile, the first information 530 may be generated via analysis of the pathological slide images 510 of the subjects. For example, the pathological slide images 510 may be analyzed via a machine learning model 520 that is pre-trained.

For example, the machine learning model 520 may be a model trained to detect a particular morphology on the basis of a mechanism of the drug. For example, when morphology matching a mechanism of a PD-L1 inhibitor is an immune cell, the machine learning model 520 may detect responsitivity of the immune cell according to administration of the PD-L1 inhibitor by analyzing the pathological slide images 510.

In addition, the machine learning model 520 may output a certain numerical value on the basis of a dataset indicating the result of the primary clinical trial, and the calculated and output numerical may also be included in the first information 530. In the above example, the machine learning model 520 may output a particular value (e.g., an inflamed score or the like) related to the immune cell by using the dataset indicating the result of the primary clinical trial.

FIG. 6 is a view illustrating an example of second information according to an embodiment.

Referring to FIG. 6, second information 630 may be generated on the basis of information 610 regarding a mechanism of a drug and information 620 regarding a plurality of candidate biomarkers. For example, the processor 110 may identify an association between a candidate biomarker and a mechanism of a drug, and may assign a priority to the candidate biomarker on the basis of the association.

A biomarker may refer to a biological element found in blood, a tissue, or the like, and may include, for example, a genome, a protein, a molecule, and the like. The biomarker may act as a sign indicating a normal process or an abnormal process, or a sign indicating a disease or a condition.

In detail, the association between the drug and the candidate biomarker, which is included in the second information 630, may be determined by at least one of an expression level of at least one genome related to the mechanism of the drug in a cancer cell, a relationship between expression locations of candidate biomarkers and a location at which the drug acts, and a genome oncogenic addiction related to the cancer cell.

Examples in which the association between the drug and the candidate biomarker is determined from the expression level of the at least one genome related to the mechanism of the drug in the cancer cell are as follows.

As an example, an expression level of a genome, which is expected to be associated with a particular drug, may be identified on the basis of The Cancer Genome Atlas (TCGA). For example, when the drug is a PD-1 inhibitor, a cytolytic score, an IFNG score, or the like may be identified as an expression level of a genome that is expected to be associated with the corresponding drug. Here, the expression level of the genome, which is expected to be associated with the drug, may be predicted from biological features extracted from a pathological slide image. Accordingly, the processor 110 may determine at least one genome having a high expression level as a biomarker having a high association with the drug from among candidate biomarkers.

As another example, on the basis of the results of analysis of genetic information of cancer patients, from among all patients, a group of patients having genomes amplified to be greater than or equal to a threshold value (e.g., 5 %) may be selected as a candidate group. From among the genomes in the candidate group, high priorities may be assigned to genomes that may have significantly higher expression levels (e.g., a false discovery rate < 1 %) in cancer cells than normal cells and have higher probabilities of being associated with the drug. In addition, a high priority may be assigned to at least one genome A having a significant difference (e.g., a false discovery rate < 1 %) in an expression level between a sample of a subject having a cancer cell in which amplification of a corresponding genome appears and a sample of a subject having a wild type of genome. Also, on the basis of the result of predicting a level B of the genome A expressed in the cancer cell from biological features extracted from a pathological slide image, a high priority may be assigned to a biomarker in which the level B of the genome A expressed in the cancer cell is best predicted, from among the biological features extracted from the pathological slide image. For example, when ANO1 gene is amplified in 6.6 % of cancer patients and expressed 2.00130154 times more in cancer patients having amplified genes than normal persons, the ANO1 gene may be determined as a biomarker, or from among the features extracted from the pathological image, a feature via which an expression level of the ANO1 genome is best predicted may be determined as a target biomarker.

Subsequently, an example in which the association between the drug and the candidate biomarker is determined from the relationship between the expression locations of the candidate biomarkers and the location at which the drug acts is as follows. For example, in relation to a mechanism of an antibody drug conjugate (ADC) drug, high priorities may be assigned to genomes in which expression positions are plasma membranes.

Subsequently, an example in which the association between the drug and the candidate biomarker is determined from the genome oncogenic addiction related to the cancer cell (e.g., whether or not the cancer cell depends on a particular genome when surviving, growing, and differentiating) is as follows. For example, when a particular genome is knocked out not to work, high priorities may be assigned to particular genomes that affect inhibition of the growth and differentiation of cancer cells.

Meanwhile, the user 30 may also randomly assign high priorities to genomes that are expected to be highly associated with a mechanism of a drug.

Referring back to FIG. 4, in operation 420, the processor 110 sets a criterion related to responsitivity to the drug, on the basis of the acquired information.

For example, the processor 110 may determine at least one of at least one biomarker from among the candidate biomarkers and a cut-off value corresponding to the at least one biomarker. In other words, the processor 110 may determine one biomarker, may determine a plurality of cut-off values of the one biomarker, may determine a plurality of biomarkers, or may determine cut-off values of the plurality of biomarkers.

Here, a determined factor (i.e., at least one biomarker or cut-off value) may be used as a criterion for distinguishing a responder and a non-responder to the drug from among subjects of the primary clinical trial.

For example, the processor 110 may set the criterion described above by using the first information, or may set the criterion described above by using both the first information and the second information.

Meanwhile, the plurality of cut-off values determined by the processor 110 may refer to quantitative information needed for the criterion for distinguishing between a responder and a non-responder to the drug from among the subjects of the primary clinical trial. For example, the plurality of cut-off values determined by the processor 110 may refer to, for example, a threshold value for one or more determined biomarkers or a value related to a threshold range. For example, the plurality of cut-off values determined by the processor 110 may be coefficient or constant information to be weighted or applied to numerical values related to the plurality of biomarkers to determine the criterion for distinguishing between the responder and the non-responder to the drug. Also, for example, the cut-off values may be weight information optimized during regression analysis on the basis of a combination of the plurality of biomarkers.

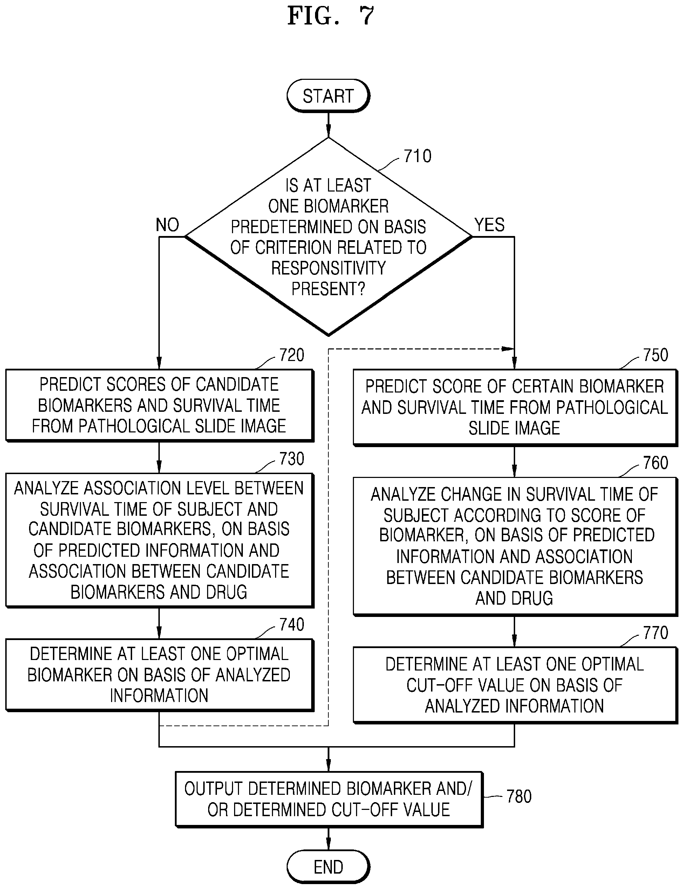

Hereinafter, an example in which the processor 110 sets the criterion related to the responsitivity to the drug is described below with reference to FIG. 7.

FIG. 7 is a flowchart illustrating an example in which a processor operates, according to an embodiment.

Referring to FIG. 7, after the process described above with reference to FIG. 6, the processor 110 may determine one biomarker (or a combination of a plurality of biomarkers) having the best outcome of a subject (e.g., when a difference in a survival time is the highest, a change in an expression level of a biological feature extracted from a pathological slide image is the greatest, or the like) compared to a control group, or at least one cut-off value for one biomarker (or a combination of a plurality of biomarkers). For example, as a criterion for extracting a responder from among subjects, the processor 110 may use a combination of biomarkers having high associations with one another (e.g., biomarkers having the same expression location, biomarkers identifiable through the same dye, and the like), or a combination of biomarkers having low associations with one another.

In operation 710, the processor 110 may identify whether or not at least one biomarker predetermined as a criterion related to responsitivity is present. In other words, the processor 110 may identify whether or not at least one biomarker, which is already known to have responsitivity to a drug, is present.

When the biomarker, which is already known to have the responsitivity to the drug, is present, the processor 110 may perform operations 750 to 770. In other words, the processor 110 may determine a cut-off value corresponding to an already known biomarker.

When the biomarker, which is already known to have the responsitivity to the drug, is not present, the processor 110 may perform operations 720 to 740. In other words, the processor 110 may select a biomarker having responsitivity to the drug.

Meanwhile, the processor 110 may continue to perform operation 750 after performing operation 740. In other words, the processor 110 may select at least one biomarker having responsitivity to the drug, and then may determine a cut-off value corresponding to the selected biomarker.

In operation 720, the processor 110 may predict scores of candidate biomarkers and survival times from a pathological slide image. For example, the processor 110 may predict a score of a candidate biomarker (e.g., an expression level of the candidate biomarker) and a survival time of a subject by analyzing the pathological slide image.

In operation 730, the processor 110 may analyze an association level between the survival time of the subject and the candidate biomarkers, on the basis of information predicted in operation 720 and an association between the candidate biomarkers and the drug. For example, the processor 110 may analyze the association level between the survival time of the subject and the candidate biomarker by considering the score of the candidate biomarker and the survival time together with second information.

In operation 740, the processor 110 may determine at least one optimal biomarker from among the candidate biomarkers, on the basis of the association level analyzed in operation 730. In other words, the processor 110 may select, from among the candidate biomarkers, at least one biomarker that significantly affects the survival time of the subject.

In operation 750, the processor 110 may predict a score of a certain biomarker and a survival time from the pathological slide image. In other words, the processor 110 may predict a score and survival time for a biomarker that is already known to have responsitivity to a drug by using a pathological slide image. Alternatively, the processor 110 may acquire the score and survival time for the biomarker, which is determined to be associated with the drug in operation 740, from data that is predicted and stored in advance in operation 720.

In operation 760, the processor 110 may analyze a change in the survival time of the subject according to the score of the biomarker, on the basis of information predicted in operation 750 and the association between the candidate biomarkers and the drug. For example, the processor 110 may analyze a relationship between the survival time of the subject and the biomarker by considering the score of the biomarker and the survival time together with the second information.

In operation 770, the processor 110 may determine at least one optimal cut-off value for the biomarker, on the basis of the result analyzed in operation 760. In other words, the processor 110 may determine at least one cut-off value determined to significantly affect the survival of the subject. When the subjects are listed according to the score of the biomarker, the processor 110 may determine a certain value as a cut-off value when a significant difference appears between survival times of subjects before and after the certain value.

In operation 780, the processor 110 may output the at least one biomarker determined in operation 740 and/or the at least one cut-off value determined in operation 770.

Referring back to FIG. 4, in operation 430, the processor 110 may generate information related to a secondary clinical trial, on the basis of the criterion set in operation 420.

For example, the information related to the secondary clinical trial may include at least one of information regarding at least one subject who is subject to the secondary clinical trial and information related to at least one biomarker to be used for design of the secondary clinical trial.

The processor 110 may select, from among a plurality of subjects, at least one subject to participate in the secondary clinical trial, on the basis of the criterion set in operation 420. For example, the processor 110 may analyze pathological slide images acquired from a plurality of subjects by using a machine learning model, and may derive a value related to a biomarker (e.g., a tumor-infiltrating lymphocytes (TIL) density value or the like). In addition, the processor 110 may select, from among the plurality of subjects, a subject to participate in the secondary clinical trial by applying a cut-off value (e.g., the cut-off value determined in operation 420) to the value related to the biomarker. For example, the processor 110 may select patients having a TIL density, which is derived by analysis of a pathological slide image and greater than or equal to the cut-off value, as subjects to participate in the secondary clinical trial.

The method and apparatus for analyzing a biomarker, according to embodiments, may increase a success rate of a clinical trial on a clinical trial phase by predicting a responder or a non-responder responding to a particular drug.

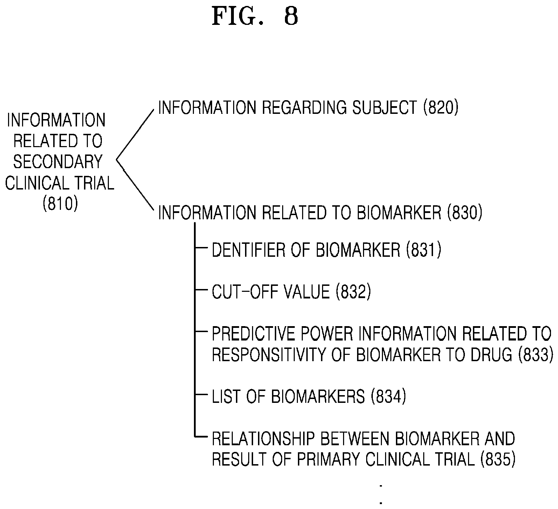

Hereinafter, an example in which the processor 110 generates information related to a secondary clinical trial is described below with reference to FIG. 8.

FIG. 8 is a view illustrating an example in which a processor operates, according to an embodiment.

Referring to FIG. 8, information 810 related to a secondary clinical trial may include information 820 regarding a subject and/or information 830 related to a biomarker. Here, the subject and the biomarker may be used to design the secondary clinical trial.

The information 820 regarding the subject may include information regarding at least one subject selected from a plurality of subjects on the basis of the result of a primary clinical trial. Here, the selected subject may be a subject to participate in the secondary clinical trial. For example, the processor 110 may analyze pathological slide images of a plurality of subjects, and may select, on the basis of the criterion described above with reference to operation 420, secondary clinical trial subjects who are expected to have high responsitivity to a drug, from among the plurality of subjects.

In addition, the information 830 related to the biomarker may include an identifier 831 of at least one biomarker, a cut-off value 832 corresponding to the at least one biomarker, predictive power information 833 related to responsitivity of the at least one biomarker to the drug (i.e., information regarding how accurately responsitivity or non-responsitivity of a subject to the drug may be predicted via at least one biomarker and/or at least one cut-off value), a list 834 in which the at least one biomarker is arranged on the basis of a certain criterion, a relationship 835 between the biomarker and the result of the primary clinical trial, and the like.

For example, the processor 110 may determine the identifier 831 and the cut-off value 832 as described above with reference to FIG. 7. Here, as described above, a biomarker and/or a cut-off value may be a criterion for distinguishing a responder to a drug from among subjects. The predictive power information 833 may include a probability capable of predicting, via the at least one biomarker and/or the at least one cut-off value, whether or not the subject responds to the drug. The list 834 may include arranging biomarkers in a certain order (e.g., a low-cost order, a preferred order of the user 30, or the like). The relationship 835 may include information regarding a biomarker that is known to have a low associated with a mechanism of the drug, but is identified to be significant according to the result of the primary clinical trial.

According to the above description, the processor 110 may set a criterion for distinguishing between a responder and a non-responder from among a plurality of subjects, on the basis of the result of the primary clinical trial and/or the result of analysis of a pathological slide image. In detail, the processor 110 may perform survival analysis in response to a score and an actual treatment result derived via pathological slide analysis, and may calculate a significant biomarker and cut-off value according to the result of the survival analysis. Accordingly, an optimal subject to participate in the secondary clinical trial may be selected from among a plurality of subjects.

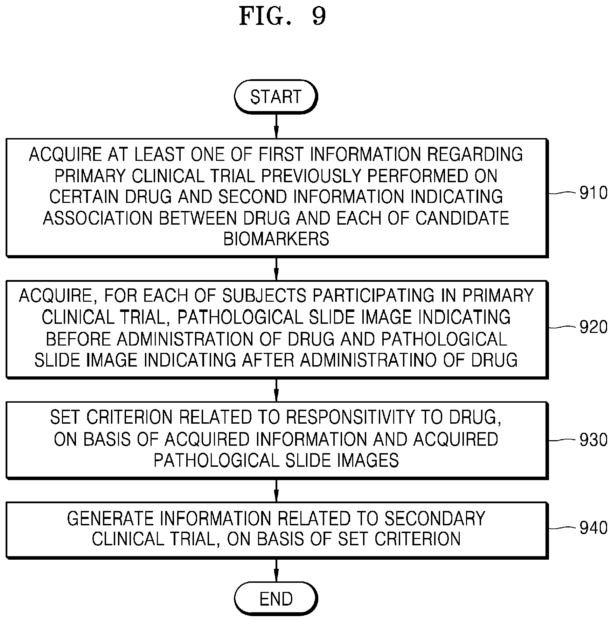

FIG. 9 is a flowchart illustrating another example of a method of analyzing a biomarker, according to an embodiment.

Referring to FIG. 9, the method of analyzing a biomarker may include operations that are processed in time series by the user terminal 10 or 100 of FIG. 1 or 3A, or the processor 110 of 3A. Therefore, although the above description of the user terminal 10 or 100 of FIG. 1 or 3A or the processor 110 of FIG. 1 is omitted below, the description may also be applied to the method of analyzing a biomarker, illustrated in FIG. 9.

Referring to FIG. 9, operations 910 and 940 correspond to operations 410 and 430. Therefore, the detailed descriptions of operations 910 and 940 are omitted hereinafter.

In operation 920, the processor 110 may acquire, for each of subjects participating in a primary clinical trial, a pathological slide image of a tissue collected before administration of a drug and a pathological slide image of a tissue collected after the administration of the drug.

In operation 930, the processor 110 may set a criterion related to responsitivity to the drug, on the basis of information acquired in operation 910 and the pathological slide images acquired in operation 920.

The processor 110 may determine a biomarker and/or a cut-off value via which a responder to the drug may be identified on the basis of changes appearing in the pathological slide images before and after the administration of the drug. For example, the processor 110 may analyze the pathological slide images before and after the administration of the drug. In addition, the processor 110 may select, as a significant biomarker, a biomarker having a great change in an expression level or indicating subjects having long predicted survival times, from among candidate biomarkers.

Meanwhile, even in a case of a biomarker that is determined to have a low association with a mechanism of the drug according to the second information, the processor 110 may select, as a significant biomarker, a biomarker that shows a great change in an expression level before and after the administration of the drug.

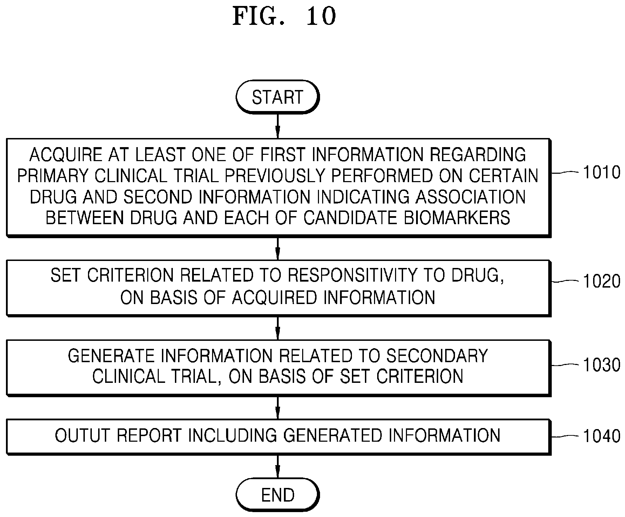

FIG. 10 is a flowchart illustrating another example of a method of analyzing a biomarker, according to an embodiment.

Referring to FIG. 10, the method of analyzing a biomarker may include operations that are processed in time series by the user terminal 10 or 100 of FIG. 1 or 3A, or the processor 110 of FIG. 3A. Therefore, although the above description of the user terminal 10 or 100 FIG. 1 or 3A or the processor 110 of FIG. 3A is omitted below, the description may also be applied to the method of analyzing a biomarker, illustrated in FIG. 10.

Referring to FIG. 10, operations 1010 to 1030 correspond to operations 410 to 430. Therefore, the detailed descriptions of operations 1010 to 1030 are omitted hereinafter.

In operation 1040, the processor 110 may output a report including information generated in operation 1030.

The processor 110 may provide the user 30 with information needed for designing a secondary clinical trial by generating the report and controlling a display to output the report. For example, the report may include information related to the secondary clinical trial described above with reference to FIG. 8, but is not limited thereto.

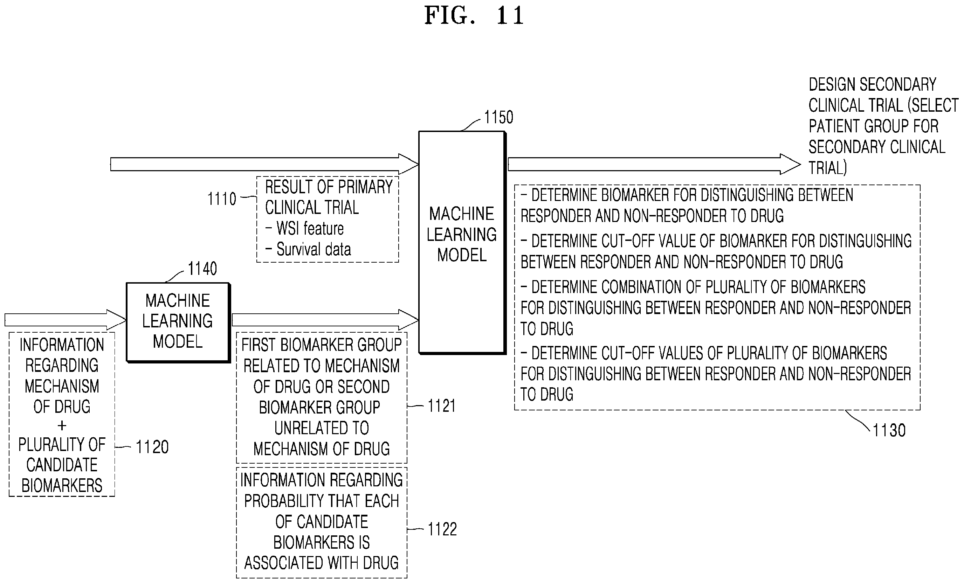

Hereinafter, examples of the operation of the user terminal 10 or 100 or the processor 110 described above with reference to FIGS. 1 to 10 are described with reference to FIGS. 11 to 14. Therefore, although the above description of the user terminal 10 or 100 or the processor 110 described above with reference to FIGS. 1 to 10 is omitted below, the description may also be applied to examples described below with reference to FIGS. 11 to 14.

FIG. 11 is a diagram illustrating an example in which a processor operates, according to an embodiment.

FIG. 11 illustrates an example in which the processor 110 sets, on the basis of first information 1110 and second information 1120, a criterion 1130 related to responsitivity to a drug. For example, the first information 1110 may refer to the result of a primary clinical trial, and may include biological features, survival data, and the like appearing in a pathological slide image. In addition, the second information 1120 may include information regarding a mechanism of the drug, a plurality of candidate biomarkers, and the like.

For example, the processor 110 may classify 1121, by using a machine learning model 1140, the plurality of candidate biomarkers according to whether or not the plurality of candidate biomarkers are related to the mechanism of the drug. In addition, the processor 110 may also acquire, by using the machine learning model 1140, a probability 1122 that each of the biomarkers is associated with the drug. Meanwhile, FIG. 11 illustrates that the processor 110 acquires additional information (e.g., 1121 and 1122) by using the second information 1120, but the present disclosure is not limited thereto. In other words, the second information 1120 may include the additional information (e.g., 1121 and 1122) in advance. In addition, detailed examples of the first information 1110 and the second information 1120 (in some cases, including the additional information (e.g., 1121 and 1122)) are as described above with reference to FIGS. 5 and 6.

The processor 110 may set, by using a machine learning model 1150, the criterion 1130 related to the responsitivity to the drug from the first information 1110 and the second information 1120 (in some cases, including the additional information (e.g., 1121 1122)). For example, the criterion 1130 may include at least one biomarker and/or at least one cut-off value for distinguishing between a responder and a non-responder to the drug. A detailed example in which the criterion 1130 is set is as described above with reference to FIG. 7.

Accordingly, the processor 110 may generate information related to a secondary clinical trial on the basis of the set criterion 1130, and the user 30 may design the secondary clinical trial on the basis of the generated information. For example, the information related to the secondary clinical trial may include at least one of information regarding at least one subject who is subject to the secondary clinical trial and information related to at least one biomarker to be used for the design of the secondary clinical trial. A detailed example of the information related to the secondary clinical trial is as described above with reference to FIG. 8.

Here, the machine learning models 1140 and 1150 may refer to, in machine learning technology and cognitive science, statistical learning algorithms implemented on the basis of a structure of a biological neural network, or structures that execute the statistical learning algorithms.

For example, the machine learning models 1140 and 1150 may refer to models which have problem-solving abilities as nodes, which artificial neurons forming a network via a combination of synapses as in a biological neural network, are trained to repeatedly adjust weights of the synapses and reduce an error between a correct output and inferred output corresponding to a particular input. For example, the machine learning models 1140 and 1150 may include any probability models, neural network models, and the like used in an artificial intelligence learning method such as machine learning or deep learning.

For example, the machine learning models 1140 and 1150 may be implemented with multilayer nodes and multilayer perceptron (MLP) constituted by connections between the multilayer nodes. The machine learning models 1140 and 1150 according to the present embodiment may be implemented by using one of various artificial neural network model structures including MLP. For example, each of the machine learning models 1140 and 1150 may include an input layer that receives an input signal or data from the outside, an output layer that outputs an output signal or data corresponding to the input data, and at least one hidden layer that is located between the input layer and the output layer, receives a signal from the input layer, extracts a feature, and transmits the extracted feature to the output layer. The output layer may receive a signal or data from the hidden layer and output the signal or data to the outside.

Therefore, the machine learning models 1140 and 1150 may be trained to extract features of one or more objects (e.g., a cell, an object, a structure, and the like) included in a pathological slide image, may be trained to detect a tissue region within the pathological slide image, may trained to select a biomarker related to a mechanism of a drug, or may be trained to determine a biomarker and/ or a cut-off value for distinguishing between a responder and a non-responder to the drug.

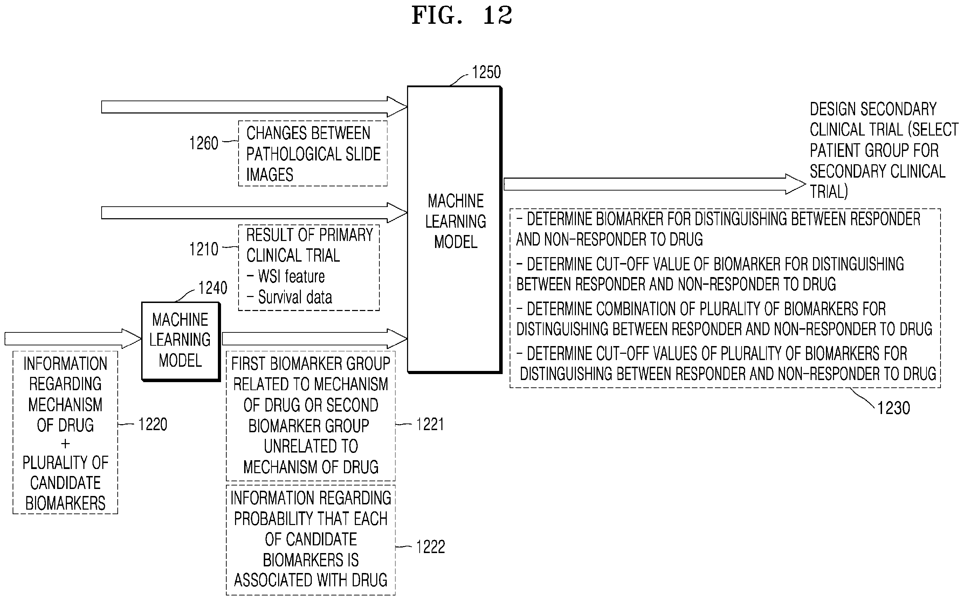

FIG. 12 is a diagram illustrating another example in which a processor operates, according to an embodiment.

Comparing FIG. 12 with FIG. 11, referring to FIG. 12, the processor 110 may set, by using a machine learning model 1250, a criterion 1230 related to responsitivity to a drug by considering not only first information 1210 and second information 1220, but also a change 1260 between pathological slide images indicating before and after administration of the drug. An example in which the processor 110 further considers the change 1260 between the pathological slide images indicating before and after the administration of the drug when setting the criterion 1230 is as described above with reference to FIG. 9.

Meanwhile, detailed examples of the first information 1210, the second information 1220, additional information 1221 and 1222, the criterion 1230, and the machine learning models 1240 and 1250 are as described above with reference to FIG. 11, and thus, detailed descriptions thereof are omitted below.

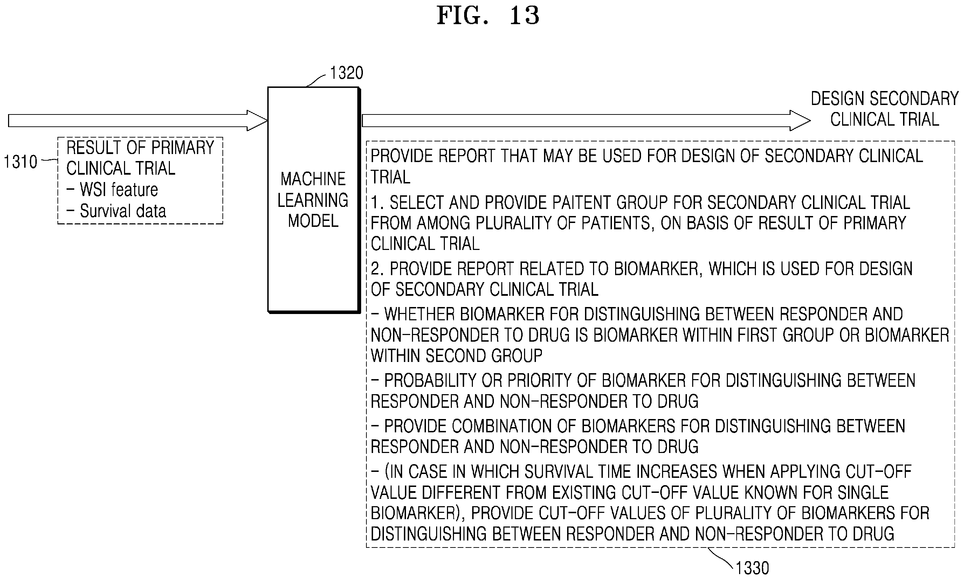

FIG. 13 is a diagram illustrating another example in which a processor operates, according to an embodiment.

Comparing FIG. 13 with FIG. 11, referring to FIG. 13, the processor 110 may set, from first information 1310, a criterion related to responsitivity to a drug by using a machine learning model 1320. In addition, the processor 110 may generate a report 1330 including information related to a secondary clinical trial, which is generated according to the set criterion. An example in which the processor 110 generates the report 1330 is as described above with reference to FIG. 10.

Meanwhile, detailed examples of the first information 1310, the information related to the secondary clinical trial, which is included in the report 1330, and the machine learning model 1320 are as described above with reference to FIG. 11, and thus, detailed descriptions thereof are omitted below.

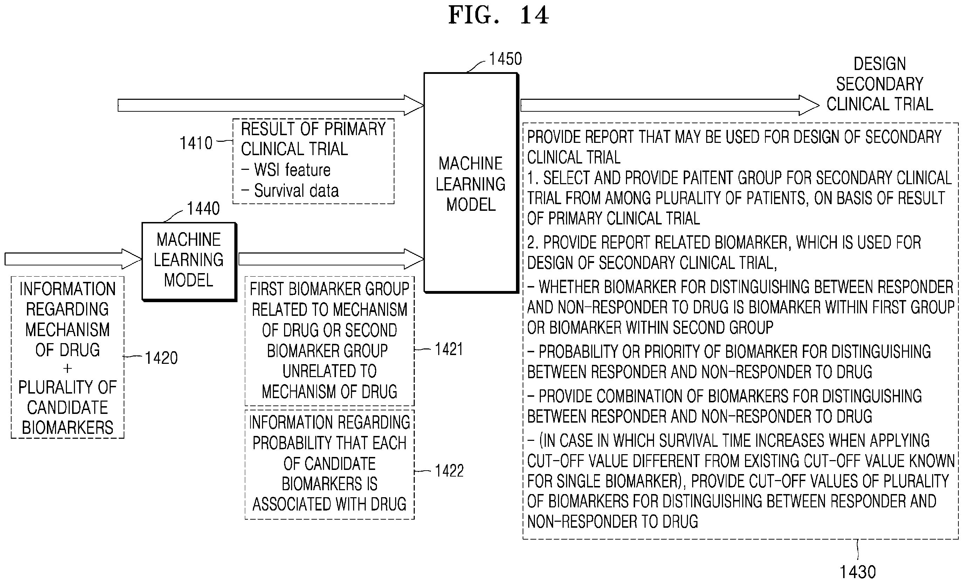

FIG. 14 is a diagram illustrating another example in which a processor operates, according to an embodiment.

FIG. 14 illustrates an example of a combination of the example described above with reference to FIG. 11 and the example described above with reference to FIG. 13. In detail, the processor 110 may set, by using a machine learning model 1450, a criterion related to responsitivity to a drug from first information 1410 and second information 1420 (in some cases, including additional information 1421, 1422). In addition, the processor 110 may generate a report 1430 including information related to a secondary clinical trial, which is generated according to the set criterion. In this case, the additional information 1421 and 1422 may be generated via a machine learning model 1440, or the second information 1420 may include the additional information 1421 and 1422 in advance.

Meanwhile, detailed examples of the first information 1410, the second information 1420, the additional information 1421 and 1422, the report 1430, and the machine learning models 1440 and 1450 are as described above with reference to FIGS. 11 and 13, and thus, detailed descriptions thereof are omitted below.

According to the above description, the processor 110 may set a criterion for distinguishing between a responder and a non-responder from among a plurality of subjects, on the basis of the result of a primary clinical trial and/or the result of analysis of a pathological slide image. In detail, the processor 110 may perform survival analysis in response to a score and an actual treatment result derived via pathological slide analysis, and may calculate a significant biomarker and cut-off value according to the result of the survival analysis. Accordingly, an optimal subject to participate in the secondary clinical trial may be selected from among the plurality of subjects.

Meanwhile, the above-described method may be written as a program that may be executed on a computer, and may be implemented in a general-purpose digital computer that operates the program by using a computer-readable recording medium. In addition, a structure of data used in the above-described method may be recorded on a computer-readable recording medium via various means. The computer-readable recording medium may include storage media such as magnetic storage media (e.g., ROM, RAM, a USB, a floppy disk, a hard disk, and the like) and optical reading media (e.g., CD-ROM, DVD, and the like).

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope as defined by the following claims.

## Claims

1. A computing apparatus comprising:
at least one memory storing at least one program; and
at least one processor configured to, by executing the at least one program:
acquire at least one of first information regarding a primary clinical trial previously performed on a certain drug and second information indicating an association between the drug and each of candidate biomarkers;
set a criterion related to responsitivity to the drug based on the acquired information; and
generate information related to a secondary clinical trial based on the set criterion.

2. The computing apparatus of claim 1, wherein the first information includes pathological slide images of subjects of the primary clinical trial.

3. The computing apparatus of claim 1, wherein the association is determined based on at least one of an expression level of at least one genome related to a mechanism of the drug in a cancer cell, a relationship between expression locations of the candidate biomarkers and a location at which the drug acts, and a genome oncogenic addiction related to the cancer cell.

4. The computing apparatus of claim 1, wherein the processor is configured to determine at least one of at least one biomarker from among the candidate biomarkers and a cut-off value corresponding to the at least one biomarker to set the criterion related to the responsitivity to the drug.

5. The computing apparatus of claim 4, wherein the at least one biomarker or the cut-off value is used as a criterion for distinguishing between a responder and a non-responder to the drug from among subjects of the primary clinical trial.

6. The computing apparatus of claim 1, wherein the information related to the secondary clinical trial includes at least one of information regarding at least one subject of the secondary clinical trial and information related to at least one biomarker to be used for design of the secondary clinical trial.

7. The computing apparatus of claim 6, wherein the information related to the at least one biomarker includes at least one of an identifier of the at least one biomarker, a cut-off value corresponding to the at least one biomarker, predictive power information related to responsitivity of the at least one biomarker to the drug, a list in which the at least one biomarker is arranged according to a certain criterion, and a relationship between the at least one biomarker and a result of the primary clinical trial.

8. The computing apparatus of claim 1, wherein the processor is configured to:
acquire, for each of subjects of the primary clinical trial, a first pathological slide image of a tissue collected before administration of the drug and a second pathological slide image of a tissue collected after the administration of the drug; and
set the criterion related to the responsitivity to the drug based on a change between the first pathological slide image and the second pathological slide image.

9. The computing apparatus of claim 1, wherein the processor is configured to control a display to output a report including the information related to the secondary clinical trial.

10. A method of analyzing a biomarker, the method comprising:
acquiring at least one of first information regarding a primary clinical trial previously performed on a certain drug and second information indicating an association between the drug and each of candidate biomarkers;
setting a criterion related to responsitivity to the drug based on the acquired information; and
generating information related to a secondary clinical trial based on the set criterion.

11. The method of claim 10, wherein the first information includes pathological slide images of subjects of the primary clinical trial.

12. The method of claim 10, wherein the association is determined based on at least one of an expression level of at least one genome related to a mechanism of the drug in a cancer cell, a relationship between expression locations of the candidate biomarkers and a location at which the drug acts, and a genome oncogenic addiction related to the cancer cell.

13. The method of claim 10, wherein the setting includes determining at least one of at least one biomarker from among the candidate biomarkers and a cut-off value corresponding to the at least one biomarker to set the criterion related to the responsitivity to the drug.

14. The method of claim 10, wherein the information related to the secondary clinical trial includes at least one of information regarding at least one subject of the secondary clinical trial and information related to at least one biomarker to be used for design of the secondary clinical trial.

15. A non-transitory computer-readable recording medium having recorded thereon a program for executing the method of claim 10 on a computer.
